# EUROPEAN PATENT APPLICATION

(11) **EP 3 587 391 A1**
(43) Date of publication of application: **01.01.2020**
(21) Application number: 18179295.3
(22) Date of filing: 22.06.2018
(51) Int. Cl.: C07C 201/12, C07C 205/11

(54) **PROCESS FOR PREPARING NITROBENZYL BROMIDES**

(71) Applicant: BASF SE, 67056 Ludwigshafen am Rhein (DE)
(72) Inventor: SCHÄFER, Bernd, 67056 Ludwigshafen (DE); BOEHLING, Ralf, 67056 Ludwigshafen (DE); SIEGEL, Wolfgang, 67056 Ludwigshafen (DE)
(74) Representative: Reitstötter Kinzebach

(57) **Abstract**

The present inveniton relates to a process for the preparation of nitrobenzyl bromide of the formula (I), 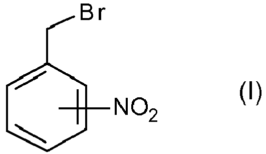 where the process comprises irradiation of a reaction mixture comprising the correspondingly substituted nitrotoluene of the formula (II), 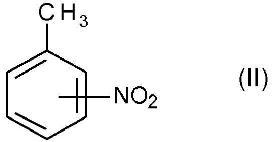 bromine and bromic acid with visible light, where the proportion of radiation having a wavelength in the range of 400 to 550 nm contributes to at least 90% of the radiation energy of the visible light used for irradiation of the reaction mixture. This process of the invention is also designated herein process A.

The invention further relates to a process for preparing 2(-pyrazol-3'-yloxymethylene)-anilide compounds, such as pyraclostrobin, via the intermediate N-carbomethoxy-N-methoxy-(2-chloromethyl)-aniline, for which 2-nitrobenzyl bromide serves as synthetic precursor.

## Description

The present inveniton relates to a process for the preparation of nitrobenzyl bromide by a bromination of the correspondingly substituted nitrotoluene. The invention further relates to a process for preparing 2(-pyrazol-3'-yloxymethylene)-anilide compounds, such as pyraclostrobin, via the intermediate N-carbomethoxy-N-methoxy-(2-chloromethyl)-aniline, for which 2-nitrobenzyl bromide serves as synthetic precursor.

Known processes for the preparation of nitrobenzyl bromides are typically based on free-radical brominations of the corresponding nitrotoluenes with bromine, where bromine is either formed in situ or added as such to the reaction mixture (the latter is exemplified in H. Cassebaum et al., Z. Chem. 1969, 9, 340). In-situ formation of bromine can be achieved by using N-bromosuccinimide (NBS) or 1,3-Dibromo-5,5-dimethylhydantoin (DBDMH) as bromine source (see, e.g., WO 2006/051476 and S. Deshpande et al., Sol. Energy 2015, 113, 332), by oxidizing hydrogen bromide (HBr) or one of its salts with an oxidant (see, e.g., EP 0 999 999), by reducing bromic acid (HBrO₃) or one of its salts with a reductant (see, e.g., S. Lan et al., Jingxi Huagong Zhongjianti 2006, 36, 24), or by comproportionation of HBr and HBrO₃ or their salts (see, e.g., S. Adimurthy et al., Green Chem. 2008, 10(2), 232). The radical brominations are typically initiated by irradiation with visible or ultraviolet light, or by the addition of a radical forming initiator, in particular organic peroxides, e.g. benzoyl peroxide, or azo compounds, e.g. azobisisobutyronitrile (AIBN).

Light initiated brominations in the presence of a bromate salt are known from the following references.

B. Yi et al., Yingyong Huaxue 2001, 18(6), 501, describe the bromination of 3-nitrotoluene under incandescent light illumination, where bromine is formed in situ by reducing sodium bromate with sodium hydrogensulfite.

M. Dinda et al., Environ. Sci. Technol. 2013, 47(18), 10535, describe the bromination of 4-nitrotoluene with HBr and sodium bromate using solar irradiation for initiating formation of free radicals.

The methods for brominating nitrotoluene to the corresponding nitrobenzyl bromide known from the prior art generally suffer from poor selectiveties and low yields, as they are often accompanied by the formation of undesired by-products, such as in particular nitrobenzal bromide (nitrobenzyl dibromide) and nitrobenzaldehyde. In addition, many of these methods are also hampered by inefficient usage of reagents, high energy consumption and/or tedious work-up procedures.

It is the object of the present invention to provide an economically attractive and technically feasible process that allows the preparation of nitrobenzyl bromide, in particular 2-nitrobenylbromide, by converting nitrotoluene with an efficient bromination system. In addition, the process should be easy to perform and be suitable for industrial scale production.

The object is achieved by the processes described in detail below.

The present invention provides a process for preparing a nitrobenzyl bromide of the formula (I), 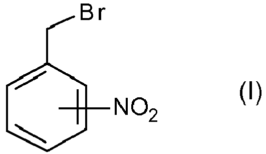 where the process comprises irradiation of a reaction mixture comprising the correspondingly substituted nitrotoluene of the formula (II), 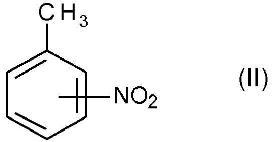 bromine and bromic acid with visible light, where the proportion of radiation having a wavelength in the range of 400 to 550 nm contributes to at least 90% of the radiation energy of the visible light used for irradiation of the reaction mixture. This process of the invention is also designated herein process A.

The process A of the present invention can be primarily considered as a free-radical bromination, where bromine serves as bromination reagent and the formation of free radicals is initiated by exposure to visible light in the wavelength range of predominately 400 to 550 nm. Process A additionally includes a comproportionation reaction of the hydrogen bromide (HBr), which is formed in the bromination step, with bromic acid (HBrO₃) also present in the reaction mixture. Accordingly, the conversion of process A can be represented by the following two equations i and ii:
(i) Bromination: 5 NT + 5 Br₂ → 5 NBB + 5 HBr
(ii) Comproportionation: 5 HBr + 1 HBrO₃ → 3 Br₂ + 3 H₂O
   wherein NT and NBB mean nitrotoluene and nitrobenzyl bromide, respectively.
   Thus, overall, the process A of the present invention and its stoichometry can generally be summarized by the following equation iii:
(iii) 5 NT + 2 Br₂ + 1 HBrO₃ → 5 NBB + 3 H₂O
   While frequently the bromine required for the reaction can be added to the reaction mixture as elemental bromine, the process A may also be carried out without adding bromine to the reaction mixture but instead generating it in situ. This can be achieved by the aforementioned comproportionation reaction of HBr with HBrO₃ according to equation ii. In this case the overall process A can generally be summarized by the following equation iii':
(iii') 3 NT + 2 HBr + 1 HBrO₃ → 3 NBB + 3 H₂O

The equations iii and iii' show that the process A is highly material-efficient, because all the bromine atoms added to the reaction mixture in the form of bromine and HBrO₃ or in the form of HBr and HBrO₃ are used for brominating nitrotoluene. Consequently, as also shown by the equation, water is, in principle, the only by-product of the overall reaction.

The process A of the invention is also very energy-efficient, as the visible light used for initiating the free-radical bromination is mostly limited to those wavelengths that are most suitable for this purpose, namely wavelengths in the the range of 400 to 550 nm. In addition, the overall reaction of process A requires only relatively modest reaction temperatures. Moreover, in contrast to sunlight or other sources of visible light, less side reactions will occur.

Besides its material- and ernergy-efficiency the process A of the present invention is associated with further advantages. For instance, process A allows for a high-yielding and specific conversion of nitrotoluene to the correspondingly substituted nitrobenzyl bromide. Process A is also simple and inexpensive and can be handled efficiently on an industrial scale. As a particular benefit process A provides effective means for preparing 2-nitrobenzyl bromide which is an important precursor for the corresponding fungicidally acitve carbamates.

With regard to the expression "correspondingly substituted nitrotoluene" used herein, "correspondingly substituted" means that the methyl group of the educt nitrotoluene is located, relative to the nitro group, in the same position as the bromomethyl group of the product nitrobenyzlbromide. For instance, if 4-nitrotoluene is used as educt, 4-nitrobenzyl bromide will be obtained as product.

The expression "visible light, where the proportion of radiation having a wavelength in the range of 400 to 550 nm contributes to at least 90% of the radiation energy of the visible light used for irradiation of the reaction mixture" used herein means that the visible light used for irradiating the reaction mixture may or may not comprise radiation having a wavelengh outside the range of 400 to 550 nm, as long as the radiation energy to which the reaction mixture is exposed originates to at least 90% from light within the wavelength range of 400 to 550 nm.

The nitrotoluene used in the process A for preparing nitrobenzyl bromide, i.e. the compound of the formula (I), is selected from the group comprising 2-nitrotoluene, 3-nitrotoluene and 4-nitrotoluene, and preferably is 2-nitrotoluene. Accordingly, the nitrobenzyl bromide obtained by process A is selected from the group comprising 2-nitrobenzyl bromide, 3-nitrobenzyl bromide and 4-nitrobenzyl bromide, and preferably is 2-nitrobenzyl bromide.

In a preferred embodiment of the present invention the nitrotoluene used in the process A of the present invention for the prepartion of nitrobenzyl bromide is 2-nitrotoluene and, thus, the nitrobenzyl bromide obtained by said process is 2-nitrobenzyl bromide.

The reactions of the present invention described herein are generally performed in reaction vessels customary for such reactions, the reactions being configurable in a continuous, semicontinuous or batchwise manner, as discussed in more detail below.

In general, the reactions of the invention are performed under atmospheric pressure, but may alternatively be performed under reduced or elevated pressure, as also discussed in more detail below.

The process A of the invention for preparing nitrobenzyl bromide comprises the conversion of nitrotoluene via a free-radical bromination effecting the introduction of a bromine substituent at the methyl group of nitrotoluene. Process A also comprises the conversion of HBr, which is formed in-situ as a by-product of the bromination step, into bromine via its comproportionation with HBrO₃. Both conversions are simultaneously effected in a single reaction mixture by reacting nitrotoluene with bromine in the presence of HBrO₃ under suitable reaction conditions.

HBrO₃ may be added to the reaction mixture as such, but is preferably generated in-situ by the reaction of a bromate salt with an acid. In principle any acid that is stable under the reaction conditions of process A can be employed for this purpose. However, strong acid, such as in particular mineral acids are preferred. Suitable mineral acids are typically selected from sulfuric acid, phosphoric acid, hydrochloric acid and nitric acid, in particular from sulfuric acid and phosphoric acid. The use of sulfuric acid is particularly preferred in this context.

The acid is usually added to the reaction mixture in an amount so that the molar ratio of bromate salt to the total protons releasable from the acid is generally in the range of 1:1 to 1:3, preferably in the range of 1:1.5 to 1:2.5, and in particular in the range of 1:1.8 to 1:2.2. For instance, if sulfuric acid is employed as acid, it is preferably added to the reaction mixture in an amount of 0.75 mol to 1.25 mol and in particular in an amount of 0.9 to 1.1 mol per 1 mol of bromate salt.

In general, any salt of bromic acid known in the art can be used as bromate salt in process A. However, an alkaline earth metal bromate, such as magnesium or calcium bromate, or an alkali metal bromate, such as sodium, potassium or lithium bromate, are preferrably employed as bromate salt in process A. More preferably the bromate salt is selected from the alkali metal salts of bromic acid, especially from sodium bromate and potassium bromate. According to a particular embodiment of the present invention the bromate salt used in process A is sodium bromate.

Frequently, a certain amount of bromine is initially present in the reaction mixture. The initial amount of bromine in the reaction mixture is usually in the range of 0.1 to 30 weight percent, preferably 1 to 23 weight percent, in particular 1 to 20 weight percent, based on the total weight of nitrotoluene and bromine. Accordingly, bromine is added in an amount of usually about 0.002 to 0.74 mol, preferably about 0.02 to 0.51 mol, in particular about 0.02 to 0.43 mol, per 1 mol of nitrotoluene. In a particular embodiment of the present invention bromine is added in a sub-stoichiometric amount to the reaction mixture, such as in an amount of 0.02 to 0.35, preferably in an amount of 0.03 to 0.3 and particularly in an amount of 0.04 to 0.25, in each case per 1 mol of nitrotoluene. In this context sub-stoichiometric amount of bromine means an amount below 0.4 mol relative to 1 mol of nitrotoluene, as apparent from the equation iii given above.

According to one embodiment of the present invention the major portion, e.g. more than 50 % and in particular at least 80 % of the bromine intended for the reaction of process, especially the total amount of bromine intended for the reaction of process A is charged in full to the reaction mixture prior to the initiation of the reaction. In this embodiment the use of a sub-stoichiometric amount of bromine, as defined above, is preferred. According to an alternative embodiment of the present invention the intended amount of bromine is gradually charged to the reaction mixture over the course of the reaction. In this embodiment the total amount of bromine added to reaction mixture is preferably in the range of 0.3 to 0.51 mol, more preferably 0.33 to 0.45 mol and in particular 0.35 to 0.43 mol, per 1 mol of nitrotoluene.

In this context, the molar ratio of the total amounts of bromine and bromic acid intended for the reaction of process A is preferably in the range of preferably 1.5:1 to 3:1, more preferably in the range of 1.7:1 to 2.3:1, and in particular in the range of 1.7:1 to 2.1:1 mol.

According to another embodiment of the present invention the major portion of bromine, e.g. more than 50 % and in particular at least 80 % of the bromine intended for the reaction of process, especially the total amount of bromine is not directly added to the reaction mixture, but generated in situ from HBr and HBrO₃ via the aforementioned comproportionation reaction as principally represented by equation ii. In this embodiment HBr and HBrO₃ are added to the reaction mixture in amounts so that the molar ratio of HBrO₃ to HBr is generally in the range of 1:1 to 1:3, preferably in the range of 1:1.5 to 1:2.5, and in particular in the range of 1:1.8 to 1:2.2.

In a preferred embodiment of the present invention the conversion of process A for preparing nitrobenzyl bromide is carried out in the presence of water. In this preferred embodiment the amount of water in the reaction mixture may largely vary and be e.g. in the range of 1 to 50 % by weight, in particular 2 to 30 % by weight, based on the total weight of the reaction mixture. The presence of water is particular benifical, because it allows the two reactions of process A, i.e. the bromination and the comproportionation, to be spatially separated from each other. This is because it is assumed that the bromination reaction of nitrotoluene with bromine is primarily located in the organic phase, while the comproportionation reaction between HBr and HBrO₃ is mainly located in the aqueous phase of the reaction mixture. The bromine generated by the comproportionation reaction is then transferred into the organic phase and, thus, fed into the bromination reaction, whereas HBr formed during the bromination is cycled back into the aqueous phase. In conclusion, the presence of water results in the partition of the two reactions into different sections of the reaction mixture, which facilitates smooth progression of both reactions, as detrimental interactions between them are largely avoided this way.

If the reaction mixture includes water, it is preferred that the reaction mixture is an emulsion. As a rule, a suitable emulsion can already be obtained by agitating the reaction mixture to a sufficient extent. However, in some instances it may be necessary or beneficial to add an emulsifier to the reaction mixture in order to enable the formation of a suitable emulsion. For this purpose any emusifier is in principle suitable that is known in the art to be useful for similar applications.

In a particular embodiment of the present invention the reaction of process A for preparing nitrobenzyl bromide is carried out in the absence of an organic solvent having no other function than serving as solvent for the reaction. In fact, nitrotoluene is not only a reactant of the reaction of process A, but typically also functions as solvent providing appropriate solvation of the other compounds involved in the reaction, such as in particular bromine. Thus, besides water, which is preferably included in the reaction mixture, as described herein, a specific solvent is usually not required for process A. It is, however, possible and may in some instances be beneficial to carry out the reaction of process A in the presence of an organic solvent. Suitable organic solvents are preferably those that are inert toward the reactants. If an organic solvent is employed for the reaction of process A at all, it has generally been found to be advantageous to use an aprotic organic solvent. Useful aprotic organic solvents here include, for example, aliphatic C₃-C₈-ethers, such as diethyl ether, diisopropyl ether, dibutyl ether, isobutyl methyl ether, methyl tert-butyl ether (MTBE), ethyl tert-butyl ether (ET-BE), 1,2-dimethoxyethane (DME) and diethylene glycol dimethyl ether (diglyme), halogenated aliphatic hydrocarbons such as methylene chloride, trichloromethane, dichloroethane, trichloroethane and tetrachloroethane, aliphatic hydrocarbons, such as pentane, hexane, heptane, octane and also petroleum ether, cycloaliphatic hydrocarbons, such as cyclopentane and cyclohexane, alicyclic C₃-C₆-ethers, such as tetrahydrofuran (THF), tetrahydropyran, 2-methyltetrahydrofuran, 3-methyltetrahydrofuran and 1,4-dioxane, aromatic hydrocarbons, such as benzene, chlorobenzene, anisole, toluene, the xylenes and mesitylene, and mixtures of these solvents with one another. Preferred aprotic organic solvents in this context are selected from halogenated aliphatic hydrocarbons, such as in particular methylene chloride, trichloromethane, dichloroethane, trichloroethane or tetrachloroethane, aromatic hydrocarbons, such as in particular chlorobenzene, and mixtures thereof.

In general, the reaction of process A is performed under temperature control. The reaction is typically effected in a closed or open reaction vessel with stirring apparatus. The reaction temperature of the conversion depends on several factors, such as the bromine concentration, and can be determined by the person skilled in the art in the individual case, for example by simple preliminary tests. In general, the conversion is performed at a temperature in the range from -20 to 100°C, preferably in the range from 0 to 90°C, more preferably in the range from 20 to 80°C, specifically in the range from 25 to 60°C and in particular in the range from 35 to 50°C.

The reaction of the process A of the invention for preparing nitrobenzyl bromide is typically carried at a pressure in the range of 50 mbar to 20 bar, preferably in the range of 0.5 to 10 bar and in particular in the range of 0.8 to 1.2 bar.

The reactants used in the reaction of process A can in principle be contacted with one another in any desired sequence. For example, nitrotoluene, preferably neat or, alternatively, dissolved in a solvent or in dispersed form, can be initally charged and admixed with bromine or, conversely, bromine, preferably neat or, alternatively, dissovled in a solvent, can be charged and admixed with nitrotoluene. Alternatively, these two reactants can also be added simultaneously to the reaction vessel. In case HBrO₃ is added as such to the reaction vessel, it can be charged neat or preferably in form of an aqueous solution before or after the addition of nitrotoluene or bromine or else together with one of these. In the preferred case where HBrO₃ is generated in-situ, a bromate salt is charged neat or preferably in form of an aqueous solution before or after the addition of nitrotoluene or bromine or else together with one of these. In the latter case an acid is added before, after or together with the bromate salt, either undiluted or as dissolved or dispersed in a solvent, such as in particular water.

It has been found to be appropriate to initially charge the reaction vessel with nitrotoluene, preferably neat or, alternatively, dissolved in an organic solvent, then add bromine and an aqueous solution of either a bromate salt or HBrO₃, and subsequently, in case a bromate salt is used, an acid which is employed undiluted or in form of an aqueous solution.

The reaction mixture of process A is irradiated with visible light, where the proportion of radiation having a wavelength in the range of 400 to 550 nm, preferably in the range of 420 to 520 and in particular in the range of 440 to 500 nm, contributes to at least 90 % of the radiation energy of the visible light used for irradiation of the reaction mixture. The irradiation of the reaction mixture may be started after nitrotoluene and bromine have been added, and is preferably started only after all initial components of the reaction mixture have been charged to the vessel.

Irradiation of the reaction mixture can be achieved by using any light source that emits visible light in the wavelength range of 400 to 550 nm, such that at least 90% of the radiation energy emitted by the light source are emitted in this range. Preferred are those light sources that emit predominately, to at least 90% based on the emitted energy, radiation in the wavelength range of 420 to 520 nm and in particular in the range of 440 to 500.

Accordingly, suitable light sources are for instance incandescent lamps, such as tungsten halogen lamps. Preferably light-emitting diodes (LEDs) are used as source of the visible light, as LEDs have a very defined and narrow range of light emission of frequently not more than 60 nm, in particular not more than 40 nm. In particular those LEDs having an emission maximum in the wavelength range of 450 to 500 nm, in particular in the range of 460 to 480 nm and specifically in the range of 465 to 475 nm are used as source of the visible light in the process of the present invention.

The reaction of process A can be carried out in a batch reactor, where the irradiation source is preferably either submerged in the reaction mixture or placed within a side loop of the reactor through which the reaction mixture is circulated. Alternatively, the reaction can be carried out semicontinuously, e.g. in cascaded reaction vessels, or continuously, e.g. in a flow reactor.

The work-up of the reaction mixture obtained in the reaction of process A and the isolation of nitrobenzyl bromide are effected in a customary manner, for example by an aqueous extractive work-up or by removing the solvent, for example under reduced pressure, and, if required, removal of unconsumed nitrotoluene, for example by distillation. Generally, nitrobenzyl bromide is obtainable in sufficient purity by applying such measures or a combination thereof. Thus, additional purification steps, in particular elaborated ones such as chromatography are usually not necessary. If desired, however, further purification can be effected by methods commonly used in the art.

Preferably, for work-up, the aqueous layer is removed, preferably after adding addional water to facilitate proper phase separation. The organic phase is usually washed with an aqueous solution of a base or reducing agent, such as sodium sulfite, then washed with water, optionally dried and afterwards, if required, subjected to a destillative separation in order to remove unconsumed nitrotoluene. The nitrobenzyl bromide thus isolated as product can subsequently be retained for uses or sent directly to a use, for example used in a further reaction step, or be purified further beforehand.

The aforementioned distillation step to remove unconsumed nitrotoluene may be benefical in case bromine was added to the reaction mixture only in a sub-stoichiometric amount, relative to the amount of nitrotoluene, as described above. The nitrotoluene recovered this way can be readily reused, for example by feeding it back as a reactant into the reaction of process A.

The present invention also relates to a process for the preparation of pyraclostrobin, i.e. methyl *N*-(2-{[1-(4-chlorophenyl)-1*H*-pyrazol-3-yl]oxymethyl}phenyl) *N*-methoxycarbamate, which includes the process A of the invention for preparing 2-nitrobenzyl bromide, and subsequently steps for converting 2-nitrobenzyl bromide into pyraclostrobin. The steps for converting 2-nitrobenzyl bromide into pyraclsotrobin are known to a skilled person, e.g. from WO 2011/113884.

For example, pyraclostrobin can be prepared from *N*-carbomethoxy-*N*-methoxy-2-(chloromethyl)-aniline, hereinafter "2-chloromethyl-aniline compound of formula III", by reacting this compound of formula III with1-(4-chlorophenyl)-3-hydroxyl-1*H*-pyrazole, hereinafter "phenylpyrazole compound of formula VII". The 2-chloromethyl-aniline compound of formula III can be prepared from 2-nitrobenzyl bromide by different routes, including the conversion of the nitro group of 2-nitrobenzyl bromide into a *N-*carbomethoxy-*N*-methoxy group and subsequent conversion of the bromomethyl group into a chloromethyl group. In particular, the 2-chloromethyl-aniline compound of formula III can be prepared from 2-nitrobenzyl bromide by the method described in WO 2011/113884. This method includes the following steps (a) to (e) as described hereinafter. 2-Nitrobenzyl bromide, in turn, is obtained according to process A of the present invention. In subsequent step (f) of the inventive process for preparing pyraclostrobin the 2-chloromethyl-aniline compound of formula III is converted into pyraclostrobin by reacting it with the phenylpyrazole compound of formula VII. The reaction steps (a) to (f) are conducted as described in WO 2011/113884 in detail to which full reference is made.

According to WO 2011/113884 the reaction sequence for preparing the compound of the formula III, 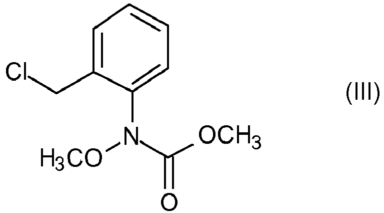 includes the following steps:
(a) reacting 2-nitrobenzyl bromide obtained from process A with a secondary amine of formula IV, 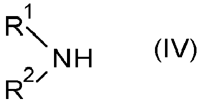 wherein
   R¹ and R² are each independently of the other selected from C₁-C₆-alkyl, C₃-C₆-alkenyl, C₂-C₆-alkoxyalkyl, C₃-C₈-cycloalkyl, phenyl and benzyl, wherein the phenyl moieties of the last two radicals mentioned may or may not carry 1, 2 or 3 substituents independently selected from halogen, C₁-C₄-alkyl and C₁-C₄-alkoxy, or
   R¹ and R² together with the nitrogen atom to which they are bound form an unsubstituted or substituted 5-, 6- or 7-membered ring that in addition to nitrogen may also contain a further heteroatom O, S or N,
   to obtain a benzylamine compound of formula V, 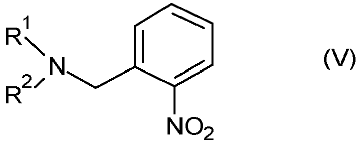 wherein R¹ and R² are as defined herein before,
(b) reducing a benzylamine compound V to obtain a hydroxylamine compound of formula Va, 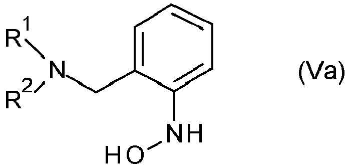
(c) acylating the hydroxylamine compound Va with a methyl haloformate to obtain a hydroxycarbamate compound of formula Vb, 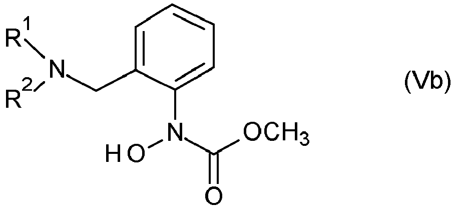
(d) methylating the hydroxycarbamate compound Vb to obtain the benzylamine compound Vc, 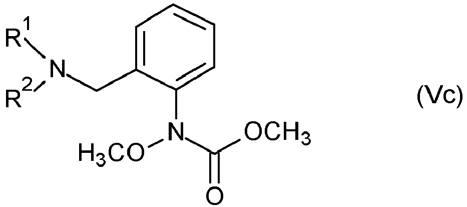
(e) reacting the benzylamine compound of formula Vc with an acyl chloride of formula VI, 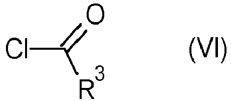 wherein:
   R³ is selected from C₁-C₆-alkoxy, C₁-C₆-haloalkoxy, C₂-C₆-alkenoxy, C₂-C₆-haloalkenoxy, C₁-C₆-alkyl, C₁-C₆-haloalkyl, C₂-C₆-alkenyl, C₂-C₆-haloalkenyl, benzyl, phenyl and benzoxy wherein the phenyl moieties of the last three radicals mentioned may or may not carry 1, 2 or 3 substituents independently selected from halogen, C₁-C₄-alkyl and C₁-C₄-alkoxy,
   to obtain the 2-chloromethyl-aniline compound of the formula III.

The terms that are used generically in the context of the process for preparing pyraclostrobin via the 2-chloromethyl-aniline compound of formula III are defined as follows:
The prefix Cₓ-C_{y} denotes the number of possible carbon atoms in the particular case.

The term "halogen" denotes in each case fluorine, bromine, chlorine or iodine, especially chlorine or bromine.

The term "C₁-C₄-alkyl" denotes a linear or branched alkyl radical comprising from 1 to 4 carbon atoms, such as methyl, ethyl, propyl, 1-methylethyl (isopropyl), butyl, 1-methylpropyl (sec-butyl), 2-methylpropyl (isobutyl) or 1,1-dimethylethyl (tert-butyl).

The term "C₁-C₆-alkyl" denotes a linear or branched alkyl radical comprising from 1 to 6 carbon atoms. Examples are, as well as the radicals specified for C₁-C₄-alkyl, pentyl, hexyl, 1-methylbutyl, 2-methylbutyl, 1-ethylpropyl, 1,1-dimethylbutyl, 1,2-dimethylbutyl, 1-ethylbutyl, 1-methylpentyl, 2-methylpetnyl, 3-methylpentyl and positional isomers thereof.

The term "cycloalkyl" denotes monocyclic saturated hydrocarbon groups having 3 to 6 (C₃-C₆-cycloalkyl), 3 to 8 (C₃-C₈-cycloalkyl) or 3 to 10 (C₃-C₁₀-cycloalkyl) carbon ring members, such as cyclopropyl, cyclobutyl, cyclopentyl, cyclohexyl, cycloheptyl, cyclooctyl, cyclononyl and cyclodecyl;

The term "C₁-C₄-haloalkyl", as used herein and in the haloalkyl units of C₁-C₄-haloalkoxy, describes straight-chain or branched alkyl groups having from 1 to 4 carbon atoms, where some or all of the hydrogen atoms of these groups have been replaced by halogen atoms. Examples thereof are chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl, pentafluoroethyl, 3,3,3-trifluoroprop-1-yl, 1,1,1-trifluoroprop-2-yl, 3,3,3-trichloroprop-1-yl, heptafluoroisopropyl, 1-chlorobutyl, 2-chlorobutyl, 3-chlorobutyl, 4-chlorobutyl, 1-fluorobutyl, 2-fluorobutyl, 3-fluorobutyl, 4-fluorobutyl and the like.

The term "C₁-C₄-alkoxy" denotes straight-chain or branched saturated alkyl groups comprising from 1 to 4 carbon atoms, which are bound via an oxygen atom to the remainder of the molecule. Examples of C₁-C₄-alkoxy are methoxy, ethoxy, n-propoxy, 1-methylethoxy (isopropoxy), n-butoxy, 1-methylpropoxy (sec-butoxy), 2-methylpropoxy (isobutoxy) and 1,1-dimethylethoxy (tert-butoxy).

The term "C₁-C₄-haloalkoxy" describes straight-chain or branched saturated haloalkyl groups comprising from 1 to 4 carbon atoms, which are bound via an oxygen atom to the remainder of the molecule. Examples thereof are chloromethoxy, bromomethoxy, dichloromethoxy, trichloromethoxy, fluoromethoxy, difluoromethoxy, trifluoromethoxy, chlorofluoromethoxy, dichlorofluoromethoxy, chlorodifluoromethoxy, 1-chloroethoxy, 1-bromoethoxy, 1-fluoroethoxy, 2-fluoroethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloroethoxy, 1,1,2,2-tetrafluoroethoxy, 1-chloro-1,2,2-trifluoroethoxy, pentafluoroethoxy, 3,3,3-trifluoroprop-1-oxy, 1,1,1-trifluoroprop-2-oxy, 3,3,3-trichloroprop-1-oxy, 1-chlorobutoxy, 2-chlorobutoxy, 3-chlorobutoxy, 4-chlorobutoxy, 1-fluorobutoxy, 2-fluorobutoxy, 3-fluorobutoxy, 4-fluorobutoxy and the like.

The term "C₂-C₆-alkoxyalkyl" refers to alkyl usually comprising 1 to 4 carbon atoms, wherein 1 carbon atom carries an alkoxy radical usually comprising 1 to 5, in particular 1 to 4, carbon atoms as defined above. Examples are CH₂OCH₃, CH₂-OC₂H₅, n-propoxymethyl, CH₂-OCH(CH₃)₂, n-butoxymethyl, (1-methylpropoxy)-methyl, (2-methylpropoxy)methyl, CH₂-OC(CH₃)₃, 2-(methoxy)ethyl, 2-(ethoxy)ethyl, 2-(n-propoxy)-ethyl, 2-(1-methylethoxy)-ethyl, 2-(n-butoxy)ethyl, 2-(1-methylpropoxy)-ethyl, 2-(2-methylpropoxy)-ethyl, 2-(1,1-dimethylethoxy)-ethyl, 2-(methoxy)-propyl, 2-(ethoxy)-propyl, 2-(n-propoxy)-propyl, 2-(1-methylethoxy)-propyl, 3-(methoxy)-propyl, 3-(ethoxy)-propyl, 3-(n-propoxy)-propyl, 3-(1-methylethoxy)-propyl, 2-(methoxy)-butyl, 2-(ethoxy)-butyl, 3-(methoxy)-butyl, 3-(ethoxy)-butyl, 4-(methoxy)-butyl, 4-(ethoxy)-butyl and the like.

The term "C₃-C₆-alkenyl" denotes monounsaturated straight-chain or branched hydrocarbon radicals having 3 to 6 carbon atoms and a double bond in any position other than the α-β-position in respect to the attachment point. Examples thereof are 2-propenyl, 2-butenyl, 3-butenyl, 1-methyl-2-propenyl, 2-methyl-2-propenyl, 2-pentenyl, 3-pentenyl, 4-pentenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-2-propenyl, 1-ethyl-2-propenyl, 2-hexenyl, 3-hexenyl, 4-hexenyl, 5-hexenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-2-butenyl, 1,2-dimethyl-3-butenyl, 1,3-dimethyl-2-butenyl, 1,3-dimethyl-3-butenyl, 2,2-dimethyl-3-butenyl, 2,3-dimethyl-2-butenyl, 2,3-dimethyl-3-butenyl, 3,3-dimethyl-2-butenyl, 1-ethyl-2-butenyl, 1-ethyl-3-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl, 1-ethyl-2-methyl-2-propenyl and the like.

The term "C₂-C₆-alkenyl" describes monounsaturated straight-chain or branched hydrocarbon radicals having 2 to 6 carbon atoms and a double bond in any position. Examples for C₂-C₆-alkenyl include those given above for C₃-C₆-alkenyl and in addition also ethenyl, 1-propenyl, 1-methylethenyl, 1-butenyl, 1-methyl-1-propenyl, 2-methyl-1-propenyl, 1-pentenyl, 1-methyl-1-butenyl, 2-methyl-1-butenyl, 3-methyl-1-butenyl, 1,2-dimethyl-1-propenyl, 1-ethyl-1-propenyl, 1-hexenyl, 1-methyl-1-pentenyl, 2-methyl-1-pentenyl, 3-methyl-1-pentenyl, 4-methyl-1-pentenyl, 1,2-dimethyl-1-butenyl, 1,3-dimethyl-1-butenyl, 1,3-dimethyl-2-butenyl, 2,3-dimethyl-1-butenyl, 3,3-dimethyl-1-butenyl, 1-ethyl-1-butenyl, 2-ethyl-1-butenyl, 1-ethyl-2-methyl-1-propenyl, and the like.

The term "C₂-C₆-haloalkenyl" denotes alkenyl radicals as mentioned above which are partially or fully substituted by one or more halogen atom as mentioned above, in particular fluorine, chlorine and bromine.

The term "C₂-C₆-alkenoxy" denotes alkenyl radicals as mentioned above which are bound via an oxygen atom to the remainder of the molecule, for example 1-ethenyloxy, 1-propenyloxy, 2-propenyloxy, 1-methylethenyloxy, 1-butenyloxy, 2-butenyloxy, 3-butenyloxy, 1-methyl-1-propenyloxy, 2-methyl-1-propenyloxy, 1-methyl-2-propenyloxy, 2-methyl-2-propenyloxy, 1-pentenyloxy, 2-pentenyloxy, 3-pentenyloxy, 4-pentenyloxy, 1-methyl-1-butenyloxy, 2-methyl-1-butenyloxy, 3-methyl-1-butenyloxy, 1-methyl-2-butenyloxy, 2-methyl-2-butenyloxy, 3-methyl-2-butenyloxy, 1-methyl-3-butenyloxy, 2-methyl-3-butenyloxy, 3-methyl-3-butenyl, 1,1-dimethyl-2-propenyloxy, 1,2-dimethyl-1-propenyloxy, 1,2-dimethyl-2-propenyloxy, 1-ethyl-1-propenyloxy, 1-ethyl-2-propenyloxy, 1-hexenyloxy, 2-hexenyloxy, 3-hexenyloxy, 4-hexenyloxy, 5-hexenyloxy, 1-methyl-1-pentenyloxy, 2-methyl-1-pentenyloxy, 3-methyl-1-pentenyloxy, 4-methyl-1-pentenyloxy, 1-methyl-2-pentenyloxy, 2-methyl-2-pentenyloxy, 3-methyl-2-pentenyloxy, 4-methyl-2-pentenyloxy, 1-methyl-3-pentenyloxy, 2-methyl-3-pentenyloxy, 3-methyl-3-pentenyloxy, 4-methyl-3-pentenyloxy, 1-methyl-4-pentenyloxy, 2-methyl-4-pentenyloxy, 3-methyl-4-pentenyloxy, 4-methyl-4-pentenyloxy, 1,1-dimethyl-2-butenyloxy, 1,1-dimethyl-3-butenyloxy, 1,2-dimethyl-1-butenyloxy, 1,2-dimethyl-2-butenyloxy, 1,2-dimethyl-3-butenyloxy, 1,3-dimethyl-1-butenyloxy, 1,3-dimethyl-2-butenyloxy, 1,3-dimethyl-3-butenyloxy, 2,2-dimethyl-3-butenyloxy, 2,3-dimethyl-1-butenyloxy, 2,3-dimethyl-2-butenyloxy, 2,3-dimethyl-3-butenyloxy, 3,3-dimethyl-1-butenyloxy, 3,3-dimethyl-2-butenyloxy, 1-ethyl-1-butenyloxy, 1-ethyl-2-butenyloxy, 1-ethyl-3-butenyloxy, 2-ethyl-1-butenyloxy, 2-ethyl-2-butenyloxy, 2-ethyl-3-butenyloxy, 1,1,2-trimethyl-2-propenyloxy, 1-ethyl-1-methyl-2-propenyloxy, 1-ethyl-2-methyl-1-propenyloxy and 1-ethyl-2-methyl-2-propenyloxy and the like.

The term "C₂-C₆-haloalkenoxy" describes alkenoxy radicals as mentioned above which are partially or fully substituted by one or more halogen atom as mentioned above, in particular fluorine, chlorine and bromine.

In the compounds of formulae IV, V, Va, Vb and Vc R¹ and R² are preferably either, independently of one another, selected from C₁-C₄-alkyl, C₂-C₄-alkoxyalkyl, C₃-C₈-cycloalkyl and benzyl, wherein the phenyl moiety of the benzyl radical may or may not carry 1, 2 or 3 substituents independently selected from halogen, C₁-C₂-alkyl and C₁-C₂-alkoxy, or R¹ and R² together with the nitrogen atom to which they are bound form a saturated or partially unsaturated 5-, 6- or 7-membered ring that in addition to nitrogen may also contain a further heteroatom O, S or N. More preferred are R¹ and R² that are either, independently of one another, selected from C₁-C₃-alkyl, C₂-C₄-alkoxyalkyl and C₃-C₈-cycloalkyl, or together with the nitrogen atom to which they are bound form a saturated 5- or 6-membered ring that in addition to nitrogen may also contain a further heteroatom O or N. Specifically, R¹ and R² are both methyl, ethyl or propyl, or together with the nitrogen atom to which they are bound form a pyrrolidinyl moiety, a piperidinyl moiety or a morpholinyl moiety.

In the compound of formula VI R³ is preferably C₂-C₆-alkoxy, C₂-C₆-haloalkoxy, C₁-C₆-alkyl, C₁-C₆-haloalkyl, phenyl or benzoxy, wherein the phenyl moieties of the last two radicals mentioned may or may not carry 1, 2 or 3 substituents independently selected from halogen, C₁-C₂-alkyl and C₁-C₂-alkoxy. R³ is more preferably C₂-C₅-alkoxy, C₂-C₅-haloalkoxy, C₁-C₄-alkyl or C₁-C₄-haloalkyl and even more preferably C₂-C₄-alkoxy or C₂-C₄-haloalkoxy. In particular R³ is ethoxy, propoxy, n-butoxy or iso-butoxy and specifically ethoxy or iso-butoxy.

The reaction steps (a) to (d) of the first reaction sequence are carried out to convert the nitro group of 2-nitrobenzyl bromide into the N-carbomethoxy-N-methoxy-amino group of the benzylamine compound of formula Vc. This conversion can be achieved by standard methods of organic chemistry, e.g. by the methods described in EP 0624155 and WO 96/01256, and is preferably effected as follows.

Step (a) is an amination reaction resulting in the formation of the tertiary benzylamine compound Va. The conversion is effected by reacting the secondary amine IV and 2-nitrobenzyl bromide, and optionally an auxiliary base, preferably in a solvent, under suitable reaction conditions, as described for instance in WO 2011/113884. The 2-nitrobenzyl bromide used in this reaction is obtained by the process A of the present invention. The optional auxiliary base is in particular selected from triethylamine, sodium carbonate and potassium carbonate.

The secondary amines IV are in particular selected from dimethylamine, diethylamine, dipropylamine, methylethylamine, methylpropylamine, ethylpropylamine, pyrrolidine, oxazolidine, dihydropyrrole, piperidine, piperazine, dihydropyridine and morpholine, and especially from dimethylamine, diethylamine, pyrrolidine, piperidine and morpholine.

In step (b) the benzylamine compound V is reduced to the corresponding N-hydroxylamine compound Va, as described with more detail in WO 2011/113884. Preferably, the conversion in step (b) is effected by a heterogeneous hydrogenation employing a platinum or palladium catalyst, whereas in either case the metal is usually supported on carbon, in the presence of an amine, as described for example in GB 1458753, US 3992395, GB 1428226, GB 1388523 and GB 1092027. Particularly preferred variants of this hydrogenation method for accomplishing step (b) are those described in WO 96/22967 and WO 99/12911. According to WO 96/22967 the amine is a morpholine compound which usually also functions as solvent, while according to WO 99/12911 the reaction is carried out in the presence of an aliphatic amine in an inert, aprotic solvent.

In step (c) the N-hydroxylamine compound Va is acylated under alkaline conditions with a methyl haloformate, in particular methyl chloroformate, according to methods known in the art to obtain a hydroxycarbamate compound of formula Vb. The acylation in step (c) is preferably carried out as follows: to a solution of the N-hydroxylamine compound Va in the aprotic organic solvent, such as preferably toluene, aliphatic C₃-C₈-ethers, e.g. tert-butyl methyl ether, or carboxylic esters, e.g. ethyl acetate, is added an aqueous solution of a base which is particularly selected from sodium hydrogen carbonate, potassium carbonate and sodium hydroxide. To the thus obtained biphasic mixture is then added the methyl haloformate, preferably at a reduced temperature of form -10 to 10 °C. The reaction is continued until complete or almost complete conversion.

In step (d) the hydroxycarbamate compound Vb is converted to the benzylamine compound Vc by exposure to a base, such as in particular sodium hydride, potassium hydride, lithium diisoproylamide or sodium bis(trimethylsilyl)amide, and reaction with a methylating agent, such as in particular methyl iodide or dimethyl sulfate, as described with more detail in WO 2011/113884. The conversion is customarily carried out in an essentially anhydrous aprotic organic solvent which is essentially anhydrous and is in particular selected from toluene, aliphatic C₃-C₈-ethers, in particular tert-butyl methyl ether, alicyclic C₃-C₆-ethers, in particular THF, and mixtures thereof.

The methylation in step (d) is preferably effected by initially treating the hydroxycarbamate compound Vb with the base. To this end, typically a solution of the hydroxycarbamate compound Vb is added to the dispersed or solved base at a temperature of preferably from -10 to 10 °C. Afterwards the methylating agent is added and the obtained mixture is kept at a temperature of preferably from 0 to 30 °C until complete or almost complete conversion.

In step (e) the benzylamine compound Vc is transferred to the 2-chloromethyl-aniline compound of formula III. The reaction is effected by reacting the benzylamine compound Vc with the acyl chloride of the formula VI preferably in a solvent under suitable reaction conditions, as described with more detail in WO 2011/113884. Step (e) can for instance be performed by analogy to WO 97/14688.

The acyl chloride VI is preferably selected from C₂-C₅-alkyl chloroformates, in particular ethyl chloroformate or iso-butyl chloroformate, and C₁-C₄-alkyl carboxylic acid chlorides, in particular acetic acid chloride, propionic acid chloride, n-butyric acid chloride or iso-butyric acid chloride.

The solvent for the conversion in step (e) is typically selected from essentially anhydrous aprotic organic solvents that are inert towards the reactants, and in particular from acetonitrile, DMF, aromatic hydrocarbons, especially toluene, halogenated aliphatic hydrocarbons, especially methylene chloride, and mixtures thereof.

The inventive process for preparing pyraclostrobin of formula VI, 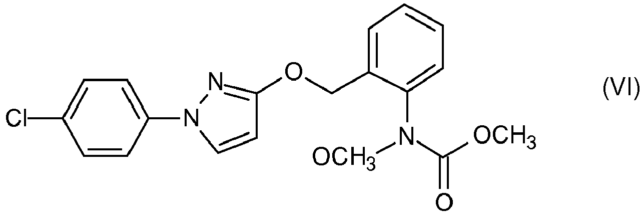 also comprises comprises the following step
(f) reacting the 2-chloromethyl-aniline compound of formula III with a phenylpyrazole compound of formula VII in the presence of a base, 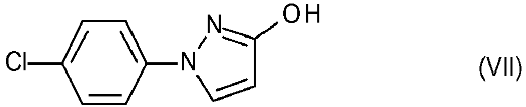
to obtain pyraclostrobin of formula VI.

Step (f) of the inventive process for preparing pyraclostrobin starting from the 2-chloromethyl-aniline compound III and the 1-phenyl-3-hydroxypyrazole compound VII is described in detail in WO 2011/113884, and is performed in a similar fashion as the etherification of a 2-nitro benzylhalide and a 3-hydroxypyrazole described in EP 0624155.

The conversion in step (f) is effected by reacting the compounds of formulae III and VII in the presence of an auxiliary base preferably in a solvent under appropriate reaction conditions.

Suitable solvents solvent for the conversion in step (f) are in particular selected from halogenated aliphatic hydrocarbons, such as methylene chloride, aromatic hydrocarbons, such as chlorobenzene or toluene, alicyclic C₃-C₆-ethers, such as THF, amides such as DMF, DMA or NMP, DMSO, acetonitrile and mixtures thereof.

The auxiliary base for the conversion in step (f) is preferably used in an amount of 0.9 to 2.5 mol, based in each case on 1 mol of compound III, and is preferably selected from sodium carbonate, potassium carbonate, potassium tert-butoxide and sodium hydroxide.

The invention is further illustrated by the following non-limiting examples.

### Examples

Hereinafter, the following abbreviations are used:
NaBrO₃ = sodium bromate
H₂SO₄ = sulfuric acid
Na₂SO₃= sodium sulfite
GC = gas chromatography
wt-% = % by weight
vol-% = % by volume

The obtained products were analyzed by HPLC on the basis of wt-%:
Device: Agilent Series 1100; column: Zorbax Phenyl-Hexyl (1.8 µm, 50 x 4.6 mm) from Agilent®; column temperature: 40°C; detector: UV-detector, λ = 202 nm, bandwidth = 4 nm; flow rate: 1,4 ml/min; injection volume: 5 µl; run time: 16 min; pressure: about 180 bar: eluents: A: water with 0.1 vol-% phosphoric acid, B: acetonitrile;
gradient:

| time (min) | eluent B (%) |
|---|---|
| 0.0 | 30 |
| 10.0 | 75 |
| 11.0 | 100 |
| 13.0 | 100 |
| 13.1 | 30 |

### Example 1: Preparation of 2-nitrobenzyl bromide

In the following expample, a closed loop reactor equipped with a monochromatic LED lamp was used. The LED lamp had the shape of an octagonal rod, which was covered on its surface with hundred LEDs (optical power: 0.96 W per LED) and which had a size about 3 cm x 20 cm. The LEDs emitted light having a wavelength of 470 nm (maximum, halfwidth of).

In the closed loop reactor 230 g (1677 mmol) of 2-nitrotoluene and 12.9 g (80.6 mmol) of bromine were mixed with a solution of 6.69 g (44.3 mmol) NaBrO₃ in 29.3 g water. 4,53 g (44,3 mmol) of H₂SO₄ (96%) were add. The obtained biphasic mixture was irradiated at 40°C with the LED lamp. When yellowing of the reaction mixture indicated completion of the reaction, 30 ml of water were added. After phase separation the organic phase was washed with a solution of 0.5 g of Na₂SO₃ in 54.5 ml of water, and again with 50 ml of water. Removal of residual water from the organic phase by evaporation under reduced pressure yielded 242.7 g of a raw product which was analyzed by HPLC. The raw product contained 17.2 wt-% of the title compound (193.5 mmol, corresponding to a yield of 94% relative to the maximum amount of bromine available over the course of the reaction). The ratio of 2-nitrobenzyl bromide to 2-nitrobenzyl dibromide was 122:1 (w/w), as also revealed by HPLC. The recovery rate of unconsumed 2-nitrotoluene was 97.3%, which was determinded as the percentage ratio over the amount of 2-nitrotoluene detected in the raw product by HPLC to the theoretical amount of unconsumed 2-nitrotoluene.

Examples 2 to 4 were carried out in analogy to Example 1 with molar ratios of bromine to 2-nitrotoluene as depicted in the following table 1 which also summerizes the results of Examples 1 to 4.

**Table 1**

| Example | molar ratio bromine to 2-NT¹⁾ | 2-NBB²⁾ | | 2-NBB²⁾: 2-NBDB⁴⁾ (w/w) | Recovery rate of 2-NT¹⁾ (%) |
|---|---|---|---|---|---|
| | | Concentration³⁾ (wt-%) | yield (%) | | |
| 1 | 0.048 | 17.2 | 96.0 | 122 : 1 | 97.3 |
| 2 | 0.095 | 31.4 | 93.9 | 60 : 1 | 96.2 |
| 3 | 0.151 | 45.3 | 91.1 | 33 : 1 | 95.4 |
| 4 | 0.215 | 55.9 | 84.0 | 17 : 1 | 93.8 |

| | | | | | |
|---|---|---|---|---|---|
| ¹⁾ 2-NT = 2-nitrotoluene ²⁾ 2-NBB = 2-nitrobenzyl bromide ³⁾ concentration in raw product ⁴⁾ 2-NBDB = 2-nitrobenzyl dibromide | | | | | |

### Example 5: Preparation of 2-nitrobenzyl bromide without addition of bromine

In a closed loop reactor containing a monochromatic LED lamp (470 nm) 244.2 g (1781 mmol) of 2-nitrotoluene, a solution of 10.1 g (67.1 mmol) NaBrO₃ in 26.2 g water were mixed with 6.83 g (67.1 mmol) of H₂SO₄ (96 %). 21.7 g (134,3 mmol) of an aqueous solution of HBr (50 wt-%) were added within 5 min. The obtained biphasic mixture was irradiated at 40 °C with a LED lamp (shape: octagonal rod covered on its surface with hundred LEDs, size: about 3 cm x 20 cm, optical power: about 96 W), which emitted light having a wavelength of 470 nm. When yellowing of the reaction mixture indicated completion of the reaction, 30 ml of water were added. After phase separation the organic phase was washed with a solution of 0.5 g of Na₂SO₃ in 54.5 ml of water, and again with 50 ml of water. Removal of residual water from the organic phase by evaporation under reduced pressure yielded 257.5 g of a raw product which was analyzed by HPLC. The raw product contained 15.5 wt-% of the title compound (184.5 mmol, corresponding to a yield of 91.6% relative to the maximum amount of bromine available over the course of the reaciton). The ratio of 2-nitrobenzyl bromide to 2-nitrobenzyl dibromide was 111:1 (w/w), as also revealed by HPLC. The recovery rate of unconsumed 2-nitrotoluene was 97%, which was determinded as the percentage ratio of the amount of 2-nitrotoluene detected in the raw product by HPLC to the theoretical amount of unconsumed 2-nitrotoluene.

## Claims

1. A process for preparing a nitrobenzyl bromide, which process comprises irradiation of a reaction mixture comprising the correspondingly substituted nitrotoluene, bromine and bromic acid with visible light, where the proportion of radiation having a wavelength in the range of 400 to 550 nm contributes to at least 90 % of the radiation energy of the visible light used for irradiation of the reaction mixture.

2. The process of claim 1, where the nitrotoluene is 2-nitrotoluene.

3. The process of claim 1 or 2, where the bromic acid is generated in situ from a bromate salt and an acid.

4. The process of claim 3, where the acid is a mineral acid, in particular sulfuric acid.

5. The process of claim 3 or 4, where the bromate salt is an alkali metal salt, in particular sodium bromate or potassium bromate.

6. The process of any one of the preceding claims, where the initial amount of bromine in the reaction mixture is within the range of 0.1 to 30 weight percent, in particular 1 to 20 weight percent, based on the total weight of nitrotoluene and bromine.

7. The process of any one of the preceding claims, where the molar ratio of bromine to bromic acid is in the range of 1.5:1 to 3:1, in particular 1.7:1 to 2.3:1.

8. The process of any one of claims 1 to 5, where at least 50% of the bromine, which is used in the reaction, is generated in situ by the reaction of hydrogen bromide with bromic acid.

9. The process of any one of the preceding claims, where the reaction is carried out in the presence of water.

10. The process of any one of the preceding claims, where the reaction is carried out in the absence of an organic solvent.

11. The process of any one of the preceding claims, where the reaction mixture is an emulsion.

12. The process of any one of the preceding claims, where the reaction is carried out at a temperature in the range of -20 to 100°C, in particular 20 to 80°C and at a pressure in the range of 50 mbar to 20 bar, in particular 0.8 to 1.2 bar.

13. The process of any one of the preceding claims, where the proportion of radiation having a wavelength in the range of 450 to 530 nm contributes to at least 90% of the radiation energy of the visible light used for irradiation of the reaction mixture.

14. The process of any one of the preceding claims, where a LED is used as source of the visible light.

15. A process for the preparation of pyraclostrobin which comprises the preparation of 2-nitrobenyzlbromide by the process of claim 2.
